(19) Europäisches Patentamt
European Patent Office
Office européen des brevets

(11) Numéro de publication : **0 060 745**
**B1**

(12) **FASCICULE DE BREVET EUROPÉEN**

(45) Date de publication du fascicule du brevet : **05.03.86**

(51) Int. Cl.⁴ : **C 07 D501/34**, A 61 K 31/545

(21) Numéro de dépôt : **82400321.4**

(22) Date de dépôt : **24.02.82**

(54) **Nouveaux dérivés antibiotiques dérivés des céphalosporines.**

(30) Priorité : **03.03.81 FR 8104243**

(43) Date de publication de la demande : **22.09.82 Bulletin 82/38**

(45) Mention de la délivrance du brevet : **05.03.86 Bulletin 86/10**

(84) Etats contractants désignés : **AT BE CH DE FR GB IT LI LU NL SE**

(56) Documents cités :
**EP-A- 0 042 154**
**FR-A- 2 321 891**
**US-A- 4 165 430**

(73) Titulaire : **SANOFI, société anonyme**
**40, Avenue George V**
**F-75008 Paris (FR)**

(72) Inventeur : **Labeeuw, Bernard**
**22 Rue Paul Eluard**
**F-34100 Montpellier (FR)**
Inventeur : **Salhi, Ali**
**639 Rue de Valène**
**F-34980 St.-Gely du Fesc (FR)**

(74) Mandataire : **Gillard, Marie-Louise et al**
**Cabinet Beau de Loménie 55, Rue d'Amsterdam**
**F-75008 Paris (FR)**

**EP 0 060 745 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne des dérivés de la famille des céphalosporines, leur procédé de préparation et leur application en thérapeutique.

Les composés selon l'invention répondent à la formule :

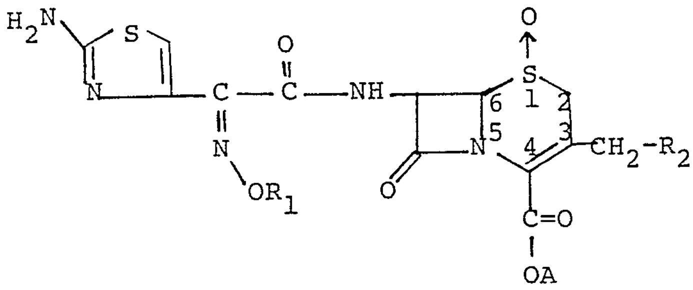

(I)

dans laquelle

le groupe

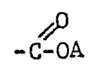

en position 4 est un radical acide, ou un sel alcalin ou alcalino-terreux ou un sel d'amine, par exemple la triéthylamine ou les éthanolamines, ou un radical ester facilement hydrolysable ou métaboliquement labile et pharmaceutiquement acceptable ;

$R_1$ représente un groupe

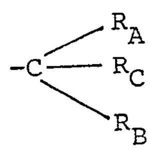

dans lequel $R_A$ et $R_B$ désignent chacun indépendamment l'hydrogène ou un groupe méthyle, ou $R_A$ et $R_B$ pris ensemble avec l'atome de carbone auquel ils sont liés forment un noyau cyclobutyle ou cyclopentyle et $R_C$ désigne l'hydrogène ou un groupe carboxylique,

$R_2$ représente un groupe

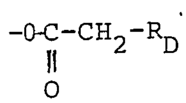

dans lequel $R_D$ représente un cycle thiazol-1,3 yl-4, éventuellement substitué en position 2 par un groupe amino, ou encore $R_2$ représente un groupe thiazolium éventuellement substitué en position 2 par un groupe amino.

Par suite de la présence dans la formule d'un groupement oxime, les composés (I) existent sous 2 formes isomères syn et anti. Les isomères syn dont l'activité thérapeutique est supérieure sont les composés préférés.

Il est entendu que les composés (I) indiqués ci-dessus peuvent exister :

— soit sous la forme indiquée dans la formule (I),

— soit sous la forme tautomère (I') :

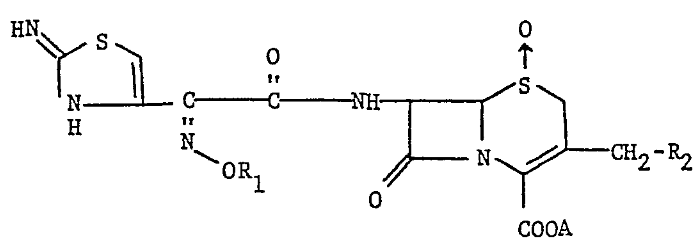

dans laquelle A, $R_1$ et $R_2$ ont les significations indiquées précédemment.

L'invention concerne également un procédé de préparation des composés de formule (I).

Ce procédé consiste à acyler tout d'abord l'amino-7 bromo-méthyl-3 céphème-3 carboxylate de

tertiobutyle-4 S-oxyde-1 (II) par l'acide (III) selon le schéma réactionnel dans lequel $R_1'$ est identique à $R_1$ ou lorsque celui-ci comporte un groupe carboxylique $R_1'$ représente l'ester t-butylique correspondant

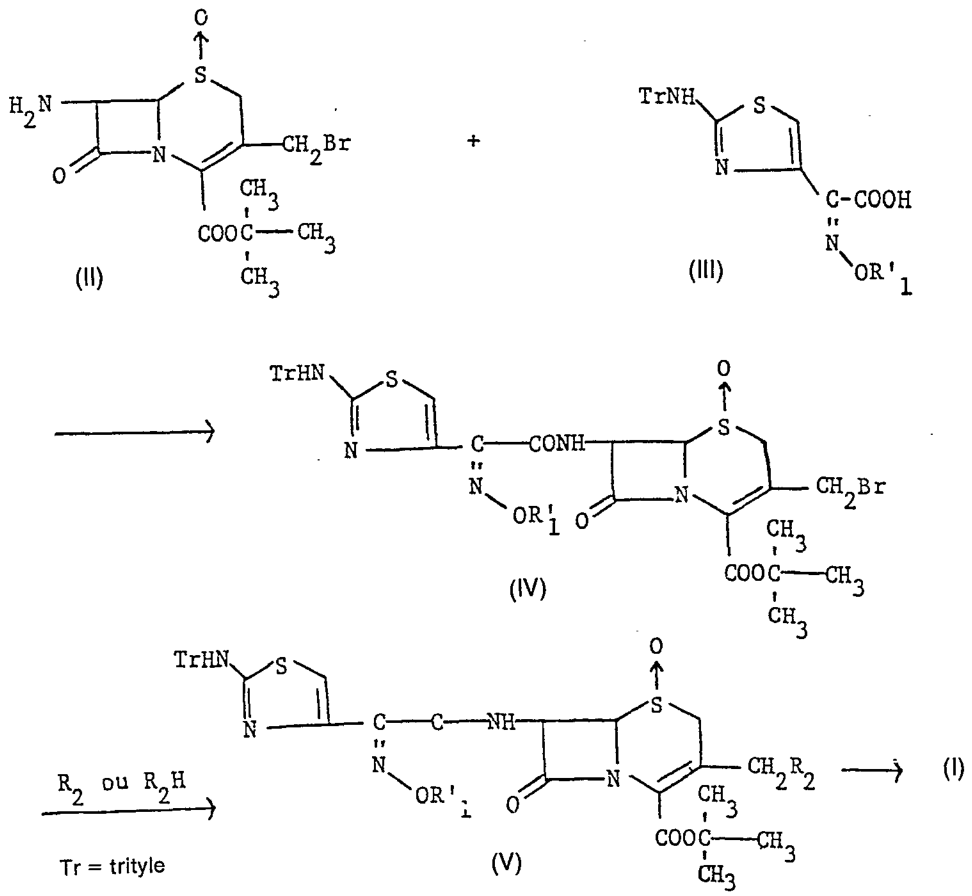


(II) + (III) → (IV) → (V) → (I)

Tr = trityle

Avant d'effectuer la réaction d'acylation, il est souhaitable de substituer le groupe amino de l'acide par un groupe protecteur facile à éliminer ultérieurement. On peut utiliser les groupes habituellement utilisés en synthèse organique pour la protection des groupes aminés et en particulier le groupe trityle.

De même, lorsque le substituant $R_1$ de l'acide (III) comporte un groupe carboxylique, il est nécessaire de transformer celui-ci en ester. On choisit de préférence un ester suffisamment labile pour pouvoir régénérer la fonction acide en fin de réaction. On utilise le plus souvent l'ester tertiobutylique.

Pour effectuer la réaction d'acylation, il est nécessaire de procéder à l'activation du groupe carboxyle du composé (III) de préférence par transformation en anhydride à l'aide d'un carbodiimide en général le dicyclohexylcarbodiimide.

La réaction d'activation est effectuée au sein d'un solvant organique convenable tel que le tétrahydrofuranne à une température comprise entre 0 et 50 °C et de préférence à température ambiante. La réaction d'activation est éventuellement facilitée par addition d'un dérivé hydroxylé tel que l'hydroxy-1 benzotriazole.

La solution du réactif d'acylation ainsi obtenue, débarrassée par filtration de la dicyclohexylurée formée, est ajoutée à une solution du composé (II) dans un solvant tel que le diméthylformamide. L'addition des 2 réactifs peut aussi s'effectuer dans l'ordre inverse.

Par action sur le composé (IV) ainsi obtenu, d'un acide ou d'une amine, on obtient le composé (V) correspondant. On opère dans un solvant convenable tel que le diméthylformamide ou le N,N-diméthylacétamide en présence d'une base comme la triéthylamine.

Enfin pour aboutir au composé (I), le groupe protecteur sur l'amine et le ou les groupes esters tertiobutyliques sont éliminés par un procédé connu, en particulier par hydrolyse en milieu acide en utilisant un acide organique tel que l'acide formique ou l'acide trifluoroacétique.

En ce qui concerne les matières premières de la réaction, les composés (II) et le composé (III) ainsi que ses dérivés dans lesquels le groupe aminé est bloqué par un groupe protecteur sont connus.

Les composés (I) de l'invention dans lesquels A est autre que H s'obtiennent à partir des composés (I) dans lesquels A est H par des réactions connues en elles-mêmes.

Ainsi, les sels minéraux sont obtenus par action sur les composés (I) dans lesquels A est H d'une base minérale telle que la soude ou la potasse ou le bicarbonate de sodium en quantité équimoléculaire ; la réaction de salification est réalisée dans un solvant tel que l'eau ou l'éthanol et le sel obtenu est isolé par évaporation de la solution.

Les sels de bases organiques sont obtenus par action, sur une solution de l'acide (I, A = H) dans un solvant ou un mélange de solvants convenables, d'une quantité équimoléculaire de la base organique. Le sel est isolé par précipitation avec l'éther.

Les esters sont obtenus par les procédés connus d'estérification ; par exemple on utilisera avantageusement l'action d'un dérivé halogéné sur un sel tel que le sel de sodium de l'acide ; on réalisera de préférence la réaction dans un solvant capable de dissoudre le dérivé acide de départ par exemple dans le diméthylformamide.

Les isomères de forme syn et anti s'obtiennent par un choix convenable des réactifs.

Les exemples suivants permettent de mieux comprendre la portée de l'invention.

Ainsi qu'il est habituel dans cette famille de composés, les produits suivant l'invention ne présentent pas de point de fusion net mais seulement des points de décomposition ne permettant pas de les caractériser.

Les produits seront donc caractérisés par leur spectre de résonance magnétique nucléaire enregistré à 60 MHz, l'étalon interne étant l'hexaméthyldisiloxane.

Les spectres sont enregistrés dans le diméthylsulfoxyde deutérié.

Les abréviations suivantes seront utilisées :

— S : singulet
— D : doublet
— D de D : doublet de doublet
— S. e. : singulet élargi
— M : multiplet
— Q : quadruplet
— AB : système AB
— J : représente la constante de couplage.

De plus les microanalyses élémentaires ont été effectuées dans chaque cas et sont en accord avec les formules indiquées.

Exemple 1

Acide [(amino-2 thiazolyl-4)-2 méthoxyimino-2 acétamido]-7 (thiazolyl-4 acétoxyméthyl)-3 céphème-3 carboxylique-4 S-oxyde-1 isomère syn.

$R_1 = CH_3$ (I)

$R_2 = -O-CO-CH_2$ 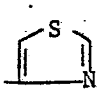

A = H

(CM 40 579).

a) [(tritylamino-2 thiazolyl-4)-2 méthoxyimino-2 acétamido]-7 bromométhyl-3 céphème-3 carboxylate de t-butyle-4 S-oxyde-1 isomère syn.

$R_1' = CH_3$ (IV)

A une solution de 4,4 g de chlorhydrate d'amino-7 bromométhyl-3 céphème-3 carboxylate de t-butyle-4 S-oxyde-1 dans 70 ml de chlorure de méthylène anhydre, on ajoute sous atmosphère d'azote, 1,5 ml de triéthylamine, 5,1 g d'acide (tritylamino-2 thiazolyl-4)-2 méthoxyimino-2 acétique isomère syn, 2,4 g de dicyclohexylcarbodiimide et 0,1 g d'hydroxy-1 benzotriazole. On agite durant 1 heure à température ambiante puis on filtre la dicyclohexylurée formée et concentre la solution à 20 ml sous vide. On chromatographie sur une colonne de gel de silice (150 g). Par élution avec le mélange hexane acétate d'éthyle 40-60 (vol/vol), on obtient, après évaporation du solvant, 4,8 g du produit attendu.

Spectre de RMN.

1 H à 8,82 ppm (N<u>H</u>—CO, D, J = 8 Hz) — 1 H à 8,70 ppm (N<u>H</u> trityle, S) — 15 H à 7,32 ppm (H aromatiques, S) — 1 H à 6,78 ppm (H thiazole, S) — 1 H à 5,79 ppm ($H_7$, D de D, $J_1$ = 8 Hz, $J_2$ = 4,5 Hz) — 1 H à 4,96 ppm ($H_6$, D, J = 4,5 Hz) — 2 H à 4,50 ppm ($CH_2$Br, S. e.) — 3 H à 3,78 ppm (NO$CH_3$, S) — 2 H à 3,77 ppm ($CH_2$S → O, S. e.) — 9 H à 1,46 ppm

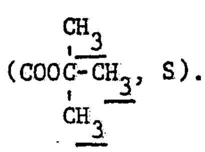

b) [(tritylamino-2 thiazolyl-4)-2 méthoxy-imino-2 acétamido]-7 (thiazolyl-4 acétoxy-méthyl)-3 céphème-3 carboxylate de t-butyle-4 S-oxyde-1 isomère syn.

$R_1' = CH_3$ (V)

$R_2$ = O-CO-$CH_2$- 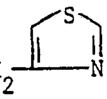

A une solution de 0,14 g d'acide thiazolyl-4 acétique dans 3,5 ml de N,N-diméthylacétamide, on ajoute 0,14 ml de triéthylamine puis 0,7 g du dérivé bromé obtenu au paragraphe a).

On agite pendant 20 heures à température ambiante puis on ajoute 50 ml d'acétate d'éthyle. On lave la solution avec 20 ml d'eau puis on sèche sur sulfate de magnésium. On évapore le solvant à siccité sous vide puis on reprend le résidu dans 5 ml de chloroforme et chromatographie la solution sur une colonne de gel de silice (30 g). On élue avec le mélange hexane-acétate d'éthyle 10-90 (vol/vol) et obtient 0,4 g du produit attendu.

Spectre de RMN.

1 H à 9,05 ppm ($H_2$, thiazole en 3, D, J = 2,5 Hz) — 2 H à 8,73 ppm (N<u>H</u> trityle, N<u>H</u>—CO, M) — 1 H à 7,55 ppm ($H_5$, thiazole en 3, D, J = 2,5 Hz) — 15 H à 7,26 ppm (H trityle, S) — 1 H à 6,80 ppm (H thiazole, S) — 1 H à 5,80 ppm ($H_7$, D, J = 4 Hz) — 1 H à 5,15 ppm (C<u>H</u>$_2$OCO—, A de AB, J = 14 Hz) — 1 H à 4,90 ppm ($H_6$, D, J = 4 Hz) — 1 H à 4,65 ppm (C<u>H</u>$_2$OCO, B de AB, J = 14 Hz) — 7 H à 3,73 ppm (C<u>H</u>$_3$ON,

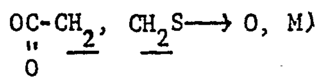

— 9 H à 1,46 ppm

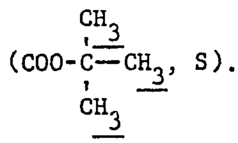

c) CM 40 579.

On dissout 0,35 g du composé obtenu ci-dessus dans 5 ml d'acide trifluoroacétique et laisse la solution à 23 °C pendant 30 minutes.

On concentre la solution sous vide jusqu'à 2 ml puis on ajoute 20 ml d'éther. On essore le précipité, lave avec de l'éther et sèche sous vide sur anhydride phosphorique.

On obtient 0,24 g du produit attendu.

Spectre de RMN.

1 H à 9,0 ppm ($H_2$, thiazole en 3, S) — 1 H à 8,90 ppm (NH—CO, D, J = 9 Hz) — 3 H à 8,40 ppm ($NH_2$, COO<u>H</u>, S. e.) — 1 H à 7,52 ppm ($H_5$, thiazole en 3, S) — 1 H à 6,88 ppm (H thiazole, S) — 1 H à 5,87 ppm ($H_7$, D de D, $J_1$ = 9 Hz, $J_2$ = 4 Hz) — 1 H à 5,20 ppm (C<u>H</u>$_2$OCO, A de AB, J = 14 Hz) — 1 H à 4,96 ppm ($H_6$, D, J = 4 Hz) — 1 H à 4,86 ppm (C<u>H</u>$_2$OCO, B de AB, J = 14 Hz) — 7 H entre 3,5 et 4 ppm (C<u>H</u>$_3$ON, C<u>H</u>$_2$S → O, OCOC<u>H</u>$_2$, M).

Exemple 2

Acide [(amino-2 thiazolyl-4)-2 carboxyméthoxyimino-2 acétamido]-7 (thiazolyl-4 acétoxyméthyl)-3 céphème-3 carboxylique-4 S-oxyde-1 isomère syn.

$R_1 = CH_2COOH$ (I)

$R_2$ = -O-CO-$CH_2$- 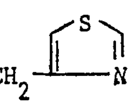

A = H
(CM 40 517).

a) [(tritylamino-2 thiazolyl-4) t-butoxycarbonyl méthoxyimino-2 acétamido]-7 bromométhyl-3 céphème-3 carboxylate de t-butyle-4 S-oxyde-1 isomère syn.

$R'_1 = CH_2$ (IV)

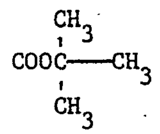

On opère comme dans l'exemple 1a) en remplaçant l'acide (tritylamino-2 thiazolyl-4)-2 méthoxyimino-2 acétique par une quantité équivalente de l'acide (tritylamino-2 thiazolyl-4)-2 t-butoxycarbonyl méthoxyimino-2 acétique isomère syn.

Spectre de RMN.

1 H à 8,75 ppm (N<u>H</u> trityle, S) — 1 H à 8,57 ppm (N<u>H</u>CO, D, J = 8,5 Hz) — 15 H à 7,28 ppm (H, aromatiques, S) — 1 H à 6,82 ppm (H thiazole, S) — 1 H à 5,84 ppm ($H_7$, D de D, $J_1$ = 8,5 Hz, $J_2$ = 4,5 Hz) — 1 H à 4,98 ppm ($H_6$, D, J = 4,5 Hz) — 4 H à 4,50 ppm (—$C\underline{H_2}$Br et $OC\underline{H_2}COO$, S) — 2 H à 3,72 ppm ($C\underline{H_2}S \rightarrow O$, S. e.) — 9 H à 1,44 ppm

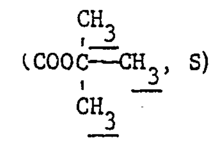

— 9 H à 1,35 ppm

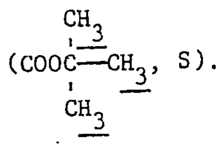

b) [(tritylamino-2 thiazolyl-4)-2 t-butoxycarbonyl méthoxyimino-2 acétamido]-7 (thiazolyl-4 acétoxyméthyl)-3 céphème-3 carboxylate de t-butyle-4 S-oxyde-1 isomère syn.

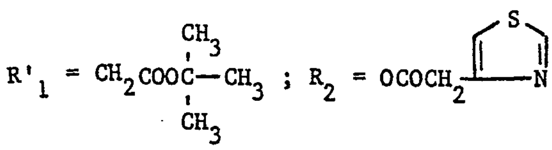 (V)

On opère comme dans l'exemple 1b) à partir du dérivé bromé obtenu ci-dessus.

Spectre de RMN.

1 H à 8,65 ppm ($H_2$, thiazole en 3, D, J = 2 Hz) — 2 H à 8,00 ppm (N<u>H</u>CO, N<u>H</u> trityle, S. e.) — 16 H à 7,25 ppm ($H_5$, thiazole en 3, H trityle, S) — 1 H à 6,73 ppm (H thiazole, S) — 1 H à 5,96 ppm ($H_7$, M) — 1 H à 5,37 ppm ($C\underline{H_2}OCO$, A de AB, J = 13 Hz) — 1 H à 4,70 ppm ($C\underline{H_2}OCO$, B de AB, J = 13 Hz) — 3 H à 4,62 ppm ($H_6$, N—O—$C\underline{H_2}$—, S. e.) — 2 H à 3,85 ppm ($OCOC\underline{H_2}$, S) — 1 H à 3,70 ppm ($CH_2S \rightarrow O$, A de AB, J = 17 Hz) — 1 H à 3,32 ppm ($C\underline{H_2}S \rightarrow O$, B de AB, J = 17 Hz) 9 H à 1,45 ppm

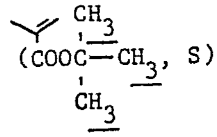

— 9 H à 1,33 ppm

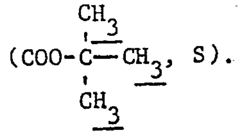

c) CM 40 517.

On opère comme dans l'exemple 1c) à partir du dérivé obtenu au paragraphe précédent.

Spectre de RMN.

1 H à 9,03 ppm ($H_2$, thiazole en 3, D, J = 2,4 Hz) — 1 H à 8,73 ppm (N<u>H</u>CO, D, J = 8,5 Hz) — 1 H à 7,53 ppm ($H_5$, thiazole en 3, D, J = 2,4 Hz) — 4 H à 7,0 ppm ($N\underline{H_2}$, 2 COO<u>H</u>, S. e.) — 1 H à 6,82 ppm (H thiazole, S) — 1 H à 5,82 ppm ($H_7$, D de D, $J_1$ = 8,5 Hz, $J_2$ = 4,5 Hz) — 1 H à 5,22 ppm ($C\underline{H_2}OCO$, A de AB, $J_{AB}$ = 14 Hz) — 1 H à 4,93 ppm ($H_6$, D, J = 4,5 Hz) — 3 H à 4,60 ppm ($OC\underline{H_2}COOH$, S et $C\underline{H_2}OCO$, B de AB, J = 14 Hz) — 2 H à 3,75 ppm ($COCH_2$ thiazole, S) — 2 H à 3,68 ppm ($C\underline{H_2}S \rightarrow O$, M).

Exemples 3 à 5

a) On opère comme dans l'exemple 1a) en remplaçant l'acide (tritylamino-2 thiazolyl-4)-2 méthoxyimino-2 acétique, par une quantité équivalente de :

— l'acide (tritylamino-2 thiazolyl-4)-2 (t-butoxy-carbonyl-2 propyl-2 oxyimino)-2 acétique ;
— l'acide (tritylamino-2 thiazolyl-4)-2 (t-butoxy-carbonyl-1-cyclobutyl-1 oxyimino)-2 acétique ;
— ou de l'acide (tritylamino-thiazolyl)-4)-2 (t-butoxy-carbonyl-1 cyclopentyl-1 oxyimino)-2 acétique.

Par le même traitement, on obtient respectivement les composés IV où :

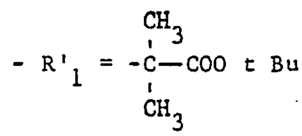

Spectre de RMN.

1 H à 8,70 ppm (N<u>H</u>-Trit, S) — 1 H à 8,07 ppm (N<u>H</u>—CO, D, J = 9 Hz) — 15 H à 7,25 ppm (H Trit, S) — 1 H à 6,72 ppm (H thiazole, S) — 1 H à 5,88 ppm ($H_7$, D de D, $J_1$ = 9 Hz, $J_2$ = 4 Hz) — 1 H à 4,96 ppm ($H_6$, D, J = 4 Hz) — 2 H à 4,50 ppm ($C\underline{H_2}Br$, AB, $J_{AB}$ = 12 Hz) — 2 H à 3,77 ppm ($C\underline{H_2}$ en 2, S. e.) — 9 H à 1,45 ppm

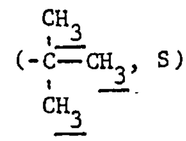

— 6 H à 1,37 ppm

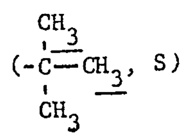

— 9 H à 1,27 ppm

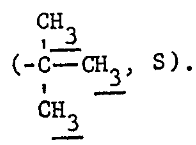

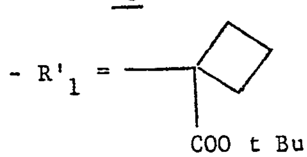

Spectre de RMN.

1 H à 7,90 ppm (N<u>H</u>CO, D, J = 9 Hz) — 15 H à 7,27 ppm ( H aromatiques, S) — 1 H à 6,97 ppm (N<u>H</u>-trityle, S. e.) — 1 H à 6,65 ppm (H thiazole, S) — 1 H à 6,18 ppm ($H_7$, D de D, J = 9 Hz, $J_2$ = 4,5 Hz) — 2 H à 3,4 ppm ($C\underline{H_2}S \rightarrow O$, S. e.) — 6 H entre 1,5 et 2,6 ppm (cyclobutyle, M) — 9 H à 1,46 ppm

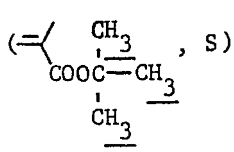

— 9 H à 1,36 ppm

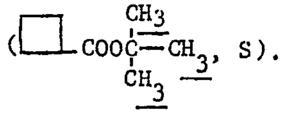

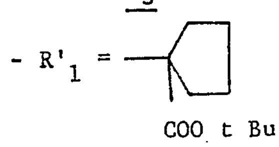

Spectre de RMN.

1 H à 7,83 ppm (N<u>H</u>CO, D, J = 9 Hz) — 15 H à 7,27 ppm (H aromatiques, S) — 1 H à 6,93 ppm (N<u>H</u>-trityle,

S. e.) — 1 H à 6,14 ppm ($H_6$, D de D, $J_1$ = 9 Hz, $J_2$ = 4,5 Hz) — 2 H à 3,5 ppm ($C\underline{H_2}S \rightarrow O$, AB, $J_{AB}$ = 17 Hz) — 8 H entre 1,3 et 2,3 ppm (cyclopentyl, M) — 9 H à 1,50 ppm

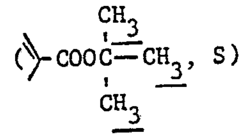

— 9 H à 1,35 ppm

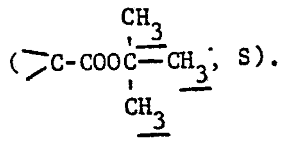

b) En faisant réagir sur les dérivés bromés obtenus ci-dessus de l'acide thiazolyl-4 acétique suivant la technique de l'exemple 1b), puis en procédant au déblocage des fonctions amine et acides ainsi qu'indiqué dans l'exemple 2c), on obtient les composés (I) suivants :

— CM 40 446

$R_1$ = 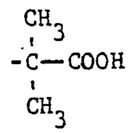 $R_2$ = 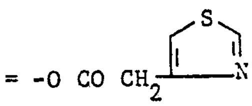

Spectre de RMN.

1 H à 9,0 ppm ($H_2$, thiazole en 3, S. e.) — 6 H à 8,5 ppm ($N\underline{H_2}$, NHCO, 2 $COO\underline{H}$, massif) — 1 H à 7,5 ppm ($H_5$, thiazole en 3, S. e.) — 1 H à 6,85 ppm (H thiazole, S. e.) — 1 H à 6,00 ppm ($H_7$, M) — 1 H à 5,27 ppm ($C\underline{H_2}OCO$, A de AB, $J_{AB}$ = 13 Hz) — 1 H à 4,97 ppm ($H_6$, M) — 1 H à 4,65 ppm ($C\underline{H_2}OCO$, B de AB, $J_{BA}$ = 13 Hz) — 4 H à 3,80 ppm ($OCOC\underline{H_2}$ et $CH_2S \rightarrow O$, M) — 6 H à 1,45 ppm

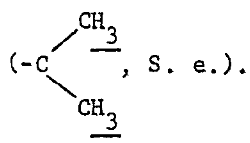

— CM 40 510

$R_1$ = 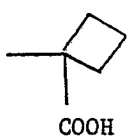 $R_2$ = 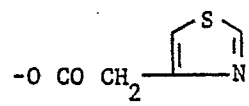

Spectre de RMN.

1 H à 8,95 ppm ($H_2$, thiazole en 3, S. e.) — 1H à 8,55 ppm ($N\underline{H}CO$, D, J = 9 Hz) — 1 H à 7,45 ppm ($H_5$, thiazole en 3, S. e.) — 4 H à 7,25 ppm (H échangeables, S. e.) — 1 H à 6,83 ppm (H thiazole, S) — 1 H à 5,96 ppm ($H_7$, D de D, $J_1$ = 9 Hz, $J_2$ = 4,5 Hz) — 1 H à 5,30 ppm ($CH_2OCO$, A de AB, $J_{AB}$ = 14 Hz) — 1 H à 5,02 ppm ($H_6$, D, J = 4,5 Hz) — 1 H à 4,76 ppm ($CH_2OCO$, B de AB, $J_{AB}$ = 14 Hz) — 2 H à 3,82 ppm ($OCOC\underline{H_2}$, S) — 2 H à 3,70 ppm ($CH_2S \rightarrow O$, S. e.) — 6 H entre 1,5 et 2,6 ppm (cyclobutyle, M).

— CM 40 511

$R_1$ = 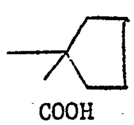 $R_2$ = 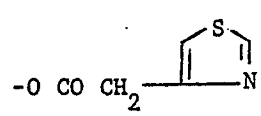

Spectre de RMN.

1 H à 9,0 ppm ($H_5$, thiazole en 3, S. e.) — 1 H à 8,46 ppm ($N\underline{H}CO$, D, J = 8,5 Hz) — 1 H à 7,50 ppm ($H_2$, thiazole en 3, S. e.) — 5 H à 7,30 ppm (H échangeables, S. e.) — 1 H à 6,84 ppm (H thiazole, S) — 1 H à 6,00 ppm ($H_7$, D de D, $J_1$ = 8,5 Hz, $J_2$ = 4,5 Hz) — 1 H à 5,20 ppm ($C\underline{H_2}OCO$, A de AB, $J_{AB}$ = 14 Hz) — 1 H à 5,00 ppm ($H_6$, D, J = 4,5 Hz) — 1 H à 4,70 ppm ($C\underline{H_2}OCO$, B de AB, $J_{BA}$ = 14 Hz) — 2 H à 3,84 ppm ($OCOC\underline{H_2}$, S) — 2 H à 3,75 ppm ($C\underline{H_2} \rightarrow O$, S. e.) — 8 H entre 1,3 et 2,4 ppm (cyclopentyle, M).

Exemple 6 et 7

Acide [(amino-2 thiazolyl-4)-2 (carboxy-1 cyclobutyl-1 oxyimino)-2 acétamido]-7 (amino-2 thiazolyl-4 acétyloxyméthyl)-3 céphème-3 carboxylique-4 S-oxyde-1 isomère syn. (CM 40 681).

$R_1$ = 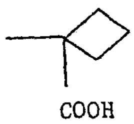 $R_2$ = -O CO $CH_2$ 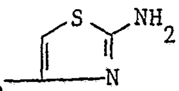 (I)

On opère comme dans l'exemple 4 en remplaçant dans la seconde étape, l'acide thiazolyl-4 acétique par de l'acide (tritylamino-2 thiazolyl-4) acétique en quantité équivalente.

Après déprotection des fonctions amines et acides, on obtient le composé attendu CM 40 681.

Spectre de RMN.

6 H entre 8 et 11 ppm (2 $N\underline{H}_2$, 2 $COO\underline{H}$, S. e.) — 1 H à 8,60 ppm ($N\underline{H}CO$, D, J = 8,5 Hz) — 1 H à 6,80 ppm (H thiazole, S) — 1 H à 6,55 ppm (H thiazole en 3, S) — 1 H à 5,95 ppm ($H_7$, D de D, $J_1$ = 8,5 Hz, $J_2$ = 4 Hz) — 1 H à 5,20 ppm ($C\underline{H}_2OCO$, A de AB, $J_{AB}$ = 13 Hz) — 1 H à 4,95 ppm ($H_6$, D, J = 4 Hz) — 1 H à 4,65 ppm ($C\underline{H}_2OCO$, B de AB, J = 13 Hz) — 4 H à 3,62 ppm ($C\underline{H}_2S \rightarrow O$, $OCOC\underline{H}_2$, M) — 6 H entre 1,5 et 2,6 ppm (cyclobutyle, M).

De même en opérant comme dans l'exemple 3 avec l'acide (trytylamino-2 thiazolyl-4) acétique, on obtient après déprotection le composé (I) :

$R_1$ = 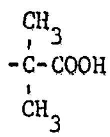 $R_2$ = -O CO $CH_2$ 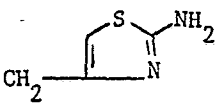

isomère syn (CM 40 733)

Spectre de RMN.

6 H entre 7 et 10 ppm (2 $N\underline{H}_2$, 2 $COO\underline{H}$, M), 1H à 8,30 ($N\underline{H}CO$, D, J = 9 Hz) — 1 H à 6,82 ppm (H thiazole, S) — 1 H à 6,42 ppm (H aminothiazole en 3,S) — 1 H à 6,00 ppm ($H_7$, D de D, $J_1$ = 9 Hz, $J_2$ = 4 Hz) — 1 H à 5,20 ppm ($C\underline{H}_2OCO$, A de AB, $J_{AB}$ = 13 Hz) — 1 H à 5,00 ppm ($H_6$, D, J = 4 Hz) — 1 H à 4,70 ppm ($C\underline{H}_2OCO$, B de AB, $J_{AB}$ = 13 Hz) — 4 H à 3,65 ppm ($C\underline{H}_2S \rightarrow O$ et

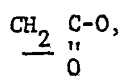 , M)

— 6 H à 1,45 ppm

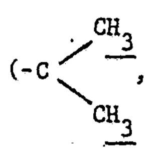 , S).

Exemples 8 et 9

Trifluoracétate de l'acide [(amino-2 thiazolyl-4)-2 (carboxy-2 propyl-2 oxyimino)-2 acétamido]-7 thiazolium méthyl-3 céphème-3 carboxylique-4 S-oxyde-1 isomère syn. (CM 40 660)

$R_1$ = 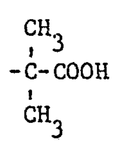 ; $R_2$ = 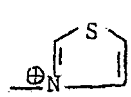 (I)

A = H

a) Bromure de [(tritylamino-2 thiazolyl-4)-2 (t-butoxycarbonyl-2 propyl-2 oxyimino)-2 acétamido]-7 thiazolium méthyl-3 céphème-3 carboxylate de t-butyle-4 S-oxyde-1 isomère syn.

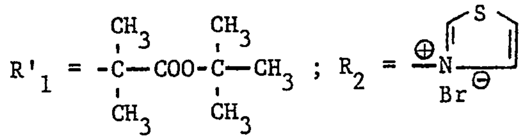 (V)

On agite pendant 24 heures à température ambiante et à l'abri de la lumière, 1 g du dérivé bromé obtenu dans l'exemple 3a) et 1,5 ml de thiazole. On ajoute de l'éther et essore le précipité qu'on lave avec de l'éther et sèche sous vide. On obtient 0,9 g du produit attendu.

Spectre de RMN.

1 H à 10,1 ppm ($H_2$, thiazole en 3, S. e.) — 1 H à 8,65 ppm (NH-trityle, S) — 2 H à 8,32 ppm ($H_4$, et $H_5$, thiazole en 3, S. e.) — 1 H à 8,15 ppm (NHCO, D, J = 8,5 Hz) — 15 H à 7,20 ppm (H aromatiques, S) — 1 H à 6,70 ppm (H thiazole, S) — 1 H à 5,85 ppm ($H_7$, D de D, $J_1$ = 8,5 Hz, $J_2$ = 4 Hz) — 2 H à 5,40 ppm

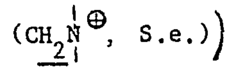

— 1 H à 5,15 ppm ($H_6$, D, J = 4 Hz) — 2 H à 3,78 ppm ($CH_2S \rightarrow O$, S. e.) — 9 H à 1,40 ppm

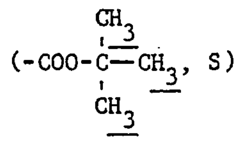

— 6 H à 1,30 ppm

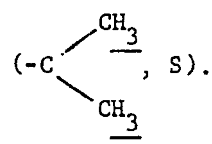

— 9 H à 1,22 ppm

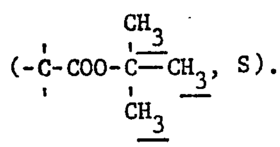

b) CM 40 660

On opère suivant l'exemple 1c).

Spectre de RMN.

1 H à 10,1 ppm ($H_2$, thiazole en 3, S. e.) — 3 H à 8,35 ppm (NHCO, $H_4$, et $H_5$; thiazole en 3, S. e.) — 1 H à 6,77 ppm (H thiazole, S) — 1 H à 6,02 ppm ($H_7$, D de D, $J_1$ = 8,5 Hz, $J_2$ = 4 Hz) — 2 H à 5,40 ppm ($CH_2$—$N^{\oplus}$—, S. e.) — 1 H à 5,0 ppm ($H_6$, D, J = 4 Hz) — 1 H à 3,72 ppm ($CH_2S \rightarrow O$, A de AB, $J_{AB}$ = 17 Hz) — 1 H à 3,55 ppm ($CH_2S \rightarrow O$, B de AB, $J_{AB}$ = 17 Hz) — 6 H à 1,42 ppm

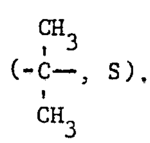

En remplaçant dans l'étape a), le thiazole par du tritylamino-2 thiazole, on obtient de la même façon, après déprotection, le composé (I) :

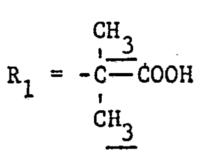

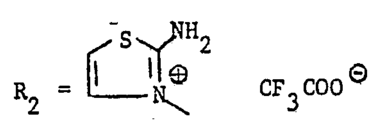

Spectre de RMN.

2 H à 9,80 ppm ($NH_2$ thiazolium) — 1 H à 8,50 ppm (NHCO, D, J = 8,5 Hz) — 2 H à 7,80 ppm ($NH_2$, S. e.) — 1 H à 7,12 ppm ($H_4$ thiazole, D, J = 4 Hz) — 1 H 7,00 ppm ($H_5$ thiazole, D, J = 4 Hz) — 1 H à 6,90 ppm (H

thiazole, S) — 1 H à 6,01 ppm ($H_7$, M) — 3 H à 5,0 ppm ($H_6$ et $C\underline{H_2}N^+$, M) — 2 H à 3,70 ppm ($C\underline{H_2}S$, AB, $J_{AB}$ = 17 Hz) — 6 H à 1,45 ppm

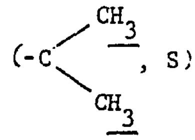

Exemples 10 et 11

En remplaçant dans l'exemple 8 le dérivé bromé par les dérivés bromés obtenus dans les exemples 4 et 5, on obtient par le même procédé les composés (I) suivants :

$- R_1 =$ 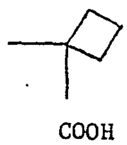 $R_2 =$ 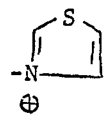 $CF_3COO^{\ominus}$ (CM 40 679)

Spectre de RMN.

1 H à 10,1 ppm ($H_2$, thiazole en 3, S) — 1 H à 8,83 ppm (N$\underline{H}$CO, D, J = 8 Hz) — 2 H à 8,40 ppm ($H_4$, et $H_5$, thiazole en 3, S) — 4 H entre 8,2 et 10 ppm ($N\underline{H_2}$, 2 COO$\underline{H}$, S. e.) — 1 H à 6,82 ppm (H thiazole, S) — 1 H à 6,01 ppm ($H_7$, M) — 2 H à 5,45 ppm

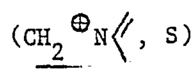

— 1 H à 5,03 ppm ($H_6$, D, J = 4 Hz) — 1 H à 3,85 ppm ($C\underline{H_2}S \rightarrow O$, A de AB, $J_{AB}$ = 17 Hz) — 1 H à 3,57 ppm ($C\underline{H_2}S \rightarrow O$, B de AB, $J_{BA}$ = 17 Hz) — 6 H entre 1,5 et 2,6 ppm (cyclobutyle, M).

$- R_1 =$ 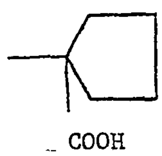 $R_2 =$ 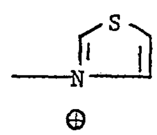 $CF_3COO^{\ominus}$ (CM 40 581)

Spectre de RMN.

1 H à 10,2 ppm ($H_2$, thiazole en 3, S) — 4 H à 9,4 ppm ($N\underline{H_2}$, 2 COO$\underline{H}$, S. e.) — 3 H à 8,45 ppm (N$\underline{H}$CO, $H_4$, et $H_5$, thiazole en 3, M) — 1 H à 6,90 ppm (H thiazole, S) — 1 H à 6,10 ppm ($H_7$, D de D, $J_1$ = 9 Hz, $J_2$ = 4 Hz) — 2 H à 5,50 ppm

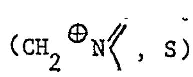

— 1 H à 5,07 ppm ($H_6$, D, J = 4 Hz) — 1 H à 3,90 ppm ($C\underline{H_2}S \rightarrow O$, A de AB, J = 17 Hz) — 1 H à 3,65 ppm ($C\underline{H_2}S \rightarrow O$, B de AB, J = 17 Hz) — 8 H entre 1,3 et 2,4 ppm (cyclopentyle, M).

Les produits de l'invention ont été étudiés en ce qui concerne leurs propriétés pharmacologiques et plus spécialement l'action bactériostatique.

L'action bactériostatique in vitro a été déterminée en milieu solide par la méthode des dilutions.

Les résultats exprimés en concentrations minimales inhibitrices (CMI — µg/ml) concernent les résultats obtenus sur les souches de Pseudomonas A 22 IP et d'Enterobacter P 99.

A titre de comparaison, on a ajouté dans le tableau les résultats avec un produit connu de structure relativement voisine : l'acide [(amino-2 thiazolyl-4)-2 méthoxyimino-2 acétamido]-7 acétoxy méthyl-3 céphème-3 carboxylique-4 S-oxyde-1 isomère syn (composé A).

CMI en µg/ml

| Produits / Souches | CM 40581 | CM 40660 | CM 40679 | CM 40762 | CM 40446 | CM 40510 | CM 40681 | CM 40733 | Composé A |
|---|---|---|---|---|---|---|---|---|---|
| Pseudomonas A 22 IP | 8 | 4 | 4 | 8 | 32 | 16 | 8 | 8 | 256 |
| Enterobacter P99 | 8 | 4 | 4 | 16 | 8 | 4 | 4 | 8 | 64 |

Ces résultats montrent une activité particulièrement intéressante des produits selon l'invention sur ces souches habituellement peu sensibles aux antibiotiques de la famille des céphalosporines.

Par ailleurs, les essais effectués sur les animaux n'ont mis en évidence aucune toxicité des produits selon l'invention. Les produits de l'invention peuvent donc être utilisés comme antibiotiques en médecine humaine ou vétérinaire. Ils peuvent être utilisés dans toutes les infections bactériennes à germes sensibles.

Les compositions pharmaceutiques sont réalisées à partir des composés (I) sous leur forme acide ou, lorsque leur solubilité est insuffisante, sous forme d'un sel.

Les compositions pharmaceutiques peuvent être solides ou liquides et se présenter par exemple sous forme de comprimés, gélules, granulés, pommades, crèmes, gels ou préparations injectables.

La posologie peut varier dans de larges proportions en particulier suivant le type et la gravité de l'infection à traiter et suivant le mode d'administration. Le plus souvent, chez l'adulte par voie injectable, elle est comprise entre 0,250 g et 4 g par jour.

A titre d'exemple de composition pharmaceutique, on peut préparer des ampoules contenant :

| | |
|---|---|
| CM 40 733 | 1 g |
| bicarbonate de sodium | 0,173 g |
| eau pour préparation injectable | 4 ml |
| ou CM 40 679 | 1 g |
| bicarbonate de sodium | 0,156 g |
| eau pour préparation injectable | 4 ml |

**Revendications** (pour les Etats contractants : BE, CH, DE, FR, GB, IT, LI, LU, NL, SE)

1. Dérivés de la famille des céphalosporines de formule

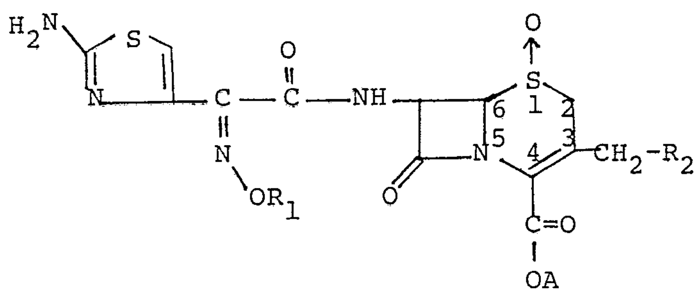

(I)

dans laquelle le groupe

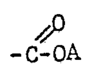

en position 4, est choisi parmi les radicaux acides (A est H), les sels alcalins, alcalino-terreux ou d'amine, telle que triéthylamine et éthanolamines, dudit acide et les radicaux esters facilement hydrolysables ou métaboliquement labiles et pharmaceutiquement acceptables,

— $R_1$ représente un groupe

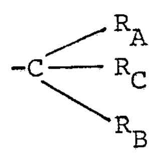

dans lequel $R_A$ et $R_B$ désignent chacun indépendamment l'hydrogène, un groupe méthyle, ou $R_A$ et $R_B$ pris ensemble avec l'atome de carbone auquel ils sont liés forment un noyau cyclobutyle ou cyclopentyle et $R_C$ désigne l'hydrogène ou un groupe carboxylique,

— $R_2$ représente un groupe

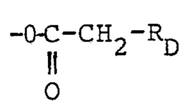

dans lequel $R_D$ représente un cycle thiazol-1,3 yl-4 éventuellement substitué en position 2 par un groupe amino, ou encore $R_2$ représente un groupe thiazolium éventuellement substitué en position 2 par un groupe amino.

2. Dérivés selon la revendication 1, caractérisés en ce qu'ils ont la forme d'isomères syn ou anti ou la forme d'un mélange de ces isomères.

3. Procédé de préparation de dérivés selon la revendication 1 dans lesquels A est H, caractérisé en ce que :

— on utilise comme produit de départ l'amino-7 bromométhyl-3 céphème-3 carboxylate de tertiobutyle-4 S-oxyde-1 pour lequel le radical amino a été protégé par exemple par le groupe trityle,

— on fait réagir ledit produit avec un acide de formule :

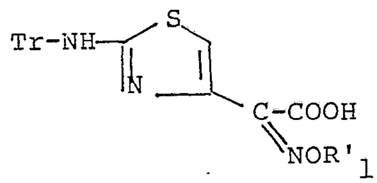

dans lequel $R_1'$ est $R_1$ ou un ester tertiobutyle de $R_1$ lorsque $R_1$ comporte une fonction carboxylique, acide dont la fonction acide a été activée, de préférence, par transformation en anhydride, la réaction ayant lieu dans un solvant, à température comprise entre 0 et 50 °C, éventuellement en présence d'un dérivé hydroxylé tel que l'hydroxy-1 benzotriazole,

— le produit en solution obtenu est ensuite mis en réaction avec un dérivé de formule $R_2H$, la réaction ayant lieu dans un solvant convenable en présence d'une base telle que la triéthylamine,

— puis, les groupes protecteurs du produit obtenu sont éliminés.

4. Procédé de préparation des produits selon la revendication 1 dans lesquels A est différent de H, caractérisé en ce que l'on prépare un produit selon la revendication 1 en utilisant le procédé selon la revendication 3 et que l'on transforme ensuite, par des moyens connus, l'hydrogène du groupe acide en position 4.

5. Médicaments utilisables comme antibiotiques en médecine humaine et vétérinaire, caractérisés en ce qu'ils contiennent, comme principe actif, un produit selon l'une des revendications 1 et 2.

6. Médicaments selon la revendication 5, caractérisés en ce que le principe actif utilisé est sous la forme syn.

7. Médicaments selon l'une des revendications 5 et 6, caractérisés en ce que le produit actif est un produit de formule (I) dans laquelle A est H, $R_1$ est

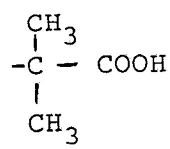

et $R_2$ est

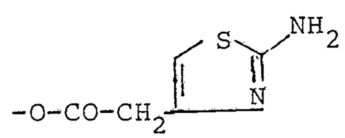

8. Médicament selon l'une des revendications 5 et 6, caractérisé en ce que le produit actif est un produit de formule (I) dans laquelle A est H, $R_1$ est

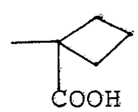,

$R_2$ est

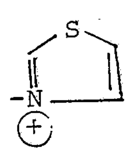

salifié avec $CF_3COO^-$.

**Revendications** (pour l'État contractant AT)

1. Procédé de préparation de dérivés de la famille des céphalosporines, de formule :

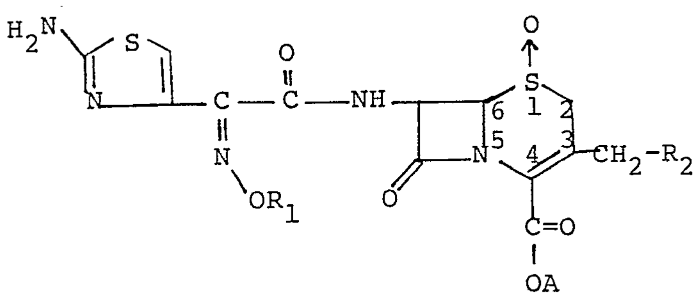

(I)

dans laquelle le groupe

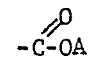

en position 4, est choisi parmi les radicaux acides (A est H), les sels alcalins, alcalino-terreux ou d'amine, telle que triéthylamine et éthanolamines, dudit acide et les radicaux esters facilement hydrolysables ou métaboliquement labiles et pharmaceutiquement acceptables,

— $R_1$ représente un groupe

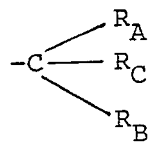

dans lequel $R_A$ et $R_B$ désignent chacun indépendamment l'hydrogène, un groupe méthyle, ou $R_A$ et $R_B$ pris ensemble avec l'atome de carbone auquel ils sont liés forment un noyau cyclobutyle ou cyclopentyle et $R_C$ désigne l'hydrogène ou un groupe carboxylique,

— $R_2$ représente un groupe

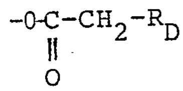

dans lequel $R_D$ représente un cycle thiazol-1,3 yl-4 éventuellement substitué en position 2 par un groupe amino ou encore $R_2$ représente un groupe thiazolium éventuellement substitué en position 2 par un groupe amino, lesdits dérivés étant sous la forme anti ou syn ou sous un mélange de ces deux formes, caractérisé en ce que :

— on utilise comme produit de départ l'amino-7 bromométhyl-3 céphème-3 carboxylate de tertiobutyle-4 S-oxyde-1 pour lequel le radical amino a été protégé par exemple par le groupe trityle,

— on fait réagir ledit produit avec un acide de formule :

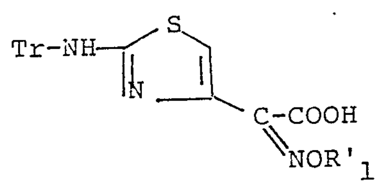

dans lequel $R_1'$ est $R_1$ ou un ester tertiobutylique de $R_1$ lorsque $R_1$ comporte une fonction carboxylique, acide dont la fonction acide a été activée, de préférence, par transformation en anhydride, la réaction ayant lieu dans un solvant, à température comprise entre 0 et 50 °C, éventuellement en présence d'un dérivé hydroxylé tel que l'hydroxy-1 benzotriazole,

— le produit en solution obtenu est ensuite mis en réaction avec un dérivé de formule $R_2H$, la réaction ayant lieu dans un solvant convenable en présence d'une base telle que la triéthylamine.

— puis, les groupes protecteurs du produit obtenu sont éliminés, après quoi on transforme éventuellement, par des moyens connus ledit acide en sel ou ester.

2. Procédé caractérisé en ce que l'on prépare, selon la revendication 1, le produit de formule (I) dans lequel A est H, $R_1$ est

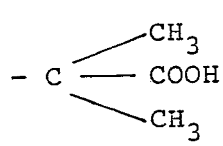

et $R_2$ est

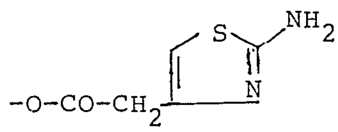

3. procédé caractérisé en ce que l'on prépare, selon la revendication 1, le produit de formule (I) dans lequel A est H, $R_1$ est

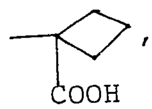

et $R_2$ est

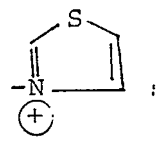

salifié avec $CF_3COO^-$.

4. Utilisation des dérivés de la famille des céphalosporines de formule (I) selon la revendication 1, pour la préparation d'un médicament antibiotique.

**Claims** (for the Contracting States : BE, CH, DE, FR, GB, IT, LI, LU, NL, SE)

1. Derivatives of the family of cephalosporins of formula :

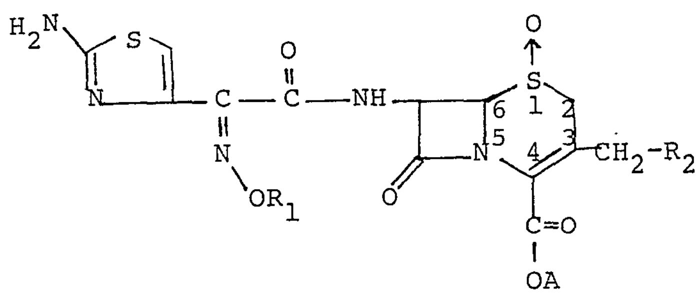

(I)

in which the

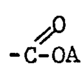

group, in 4-position, is selected from the acid radicals (A being H), the alkaline, alkaline-earth, or amine salts, such as triethylamine and ethanolamines, of said acid and the readily hydrolyzable, or metabolically labile and pharmaceutically acceptable ester radicals,

— $R_1$ is a

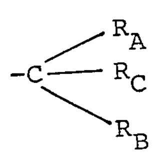

group in which $R_A$ and $R_B$ each designate independently hydrogen, a methyl group, or $R_A$ and $R_B$ taken together with the carbon atom to which they are bound, form a cyclobutyl or cyclopentyl ring, and $R_C$ designates hydrogen or a carboxylic group,

— $R_2$ is a

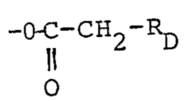

group in which $R_D$ is a 1,3-thiazol-4-yl group, optionally substituted in 2-position by an amino group, or else $R_2$ is a thiazolium group optionally substituted in 2-position by an amino group.

2. Derivatives according to claim 1, characterized in that they are in the form of syn or anti isomers or in the form of a mixture of said isomers.

3. Process for preparing derivatives according to claim 1, in which A is H, characterized in that:

— the 7-amino 3-bromomethyl 3-cephem carboxylate of 4-tert butyl 1-S-oxide, for which the amino radical has been protected, for example by the trityl group, is used as starting product,

— said product is reacted with an acid of formula :

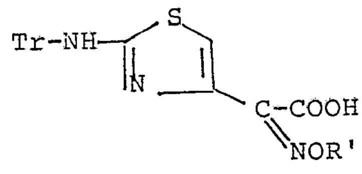

in which $R_1'$ is $R_1$ or a $R_1$ tertiobutyl ester when $R_1$ comprises an acid carboxylic function of which the acid function has been activated, preferably by conversion into anhydride, the reaction being carried out in a solvent at a temperature between 0 and 50 °C, optionally in the presence of a hydroxyl derivative such as 1-hydroxy benzotriazol,

— the resulting product in solution is reacted with a derivative of formula $R_2H$, the reaction being carried out in a suitable solvent in the presence of a base such as triethylamine,

— then, the protector groups of the resulting product are removed.

4. Process for preparing products according to claim 1, in which A is different from H, characterized in that a product according to claim 1 is prepared by using the process according to claim 3, and in that the hydrogen of the acid group in 4-position is then converted, by known means.

5. Drugs useful as antibiotics in human or veterinary medicine, characterized in that they contain, as active ingredient, a product according to any one of claims 1 and 2.

6. Drugs according to claim 5, characterized in that the used active ingredient is in the syn form.

7. Drugs according to any one of claims 5 and 6, characterized in that the active product is a product of formula (I) in which A is H, $R_1$ is

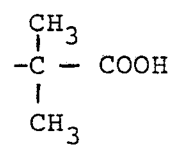

and $R_2$ is

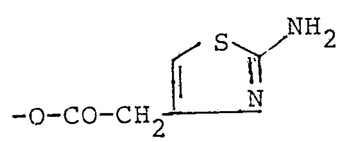

8. Drugs according to any one of claims 5 and 6, characterized in that the active product is a product of formula (I) in which A is H, $R_1$ is

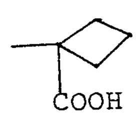

$R_2$ is

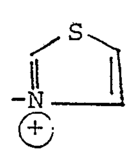

salified with $CF_3COO^-$.

**Claims** (for the Contracting State AT)

1. Process for preparing derivatives of the family of cephalosporins, of formula :

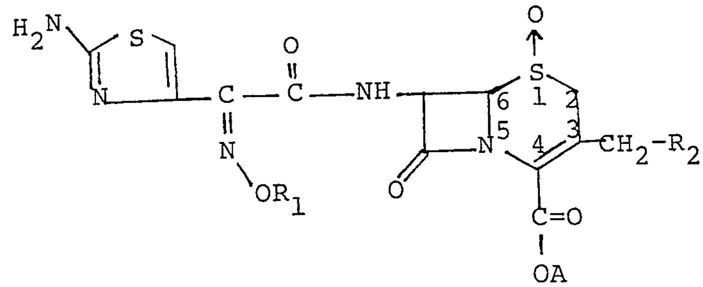 (I)

in which the

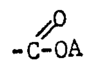

group, in 4-position, is selected from the acid radicals (A being H), the alkaline, alkaline-earth, or amine salts, such as triethylamine and ethanolamines, of said acid and the readily hydrolyzable or metabolically labile and pharmaceutically acceptable ester radicals,

— $R_1$ is a

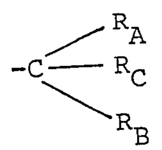

group in which $R_A$ and $R_B$ each designage independently hydrogen, a methyl group, or $R_A$ and $R_B$ taken together with the carbon atom to which they are bound, form a cyclobutyl or cyclopentyl ring, and $R_C$ designates hydrogen or a carboxylic group,

— $R_2$ is a

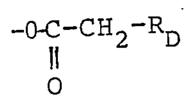

group in which $R_D$ is a 1,3 thiazol-4-yl group, optionally substituted in 2-position by an amino group, or else $R_2$ is a thiazolium group optionally substituted in 2-position by an amino group, said derivatives being in the anti or syn form or under a mixture of these two forms, characterized in that :

— the 7-amino 3-bromomethyl 3-cephem carboxylate of 4-tert butyl 1-S-oxide, for which the amino radical has been protected, for example by the trityl group, is used as starting product,

— said product is reacted with an acid of formula :

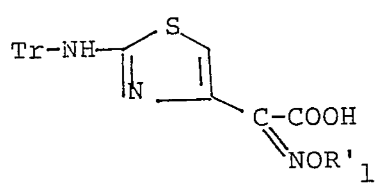

in which $R_1'$ is $R_1$ or a $R_1$ tertiobutyl ester when $R_1$ comprises an acid carboxylic function of which the acid function has been activated, preferably by conversion into anhydride, the reaction being carried out in a solvent at a temperature between 0 and 50 °C, optionally in the presence of a hydroxyl derivative such as 1-hydroxy benzotriazol,

— the resulting product in solution is reacted with a derivative of formula $R_2H$, the reaction being carried out in a suitable solvent in the presence of a base such as triethylamine,

— then, the protector groups of the resulting product are removed, said acid is afterwards optionally converted by known means, into salt or ester.

2. Process characterized in that the product of formula (I) is prepared, according to claim 1, in which A is H, $R_1$ is

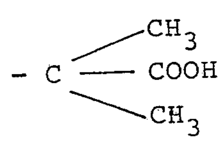

and $R_2$ is

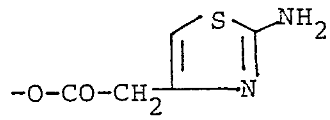

3. Process characterized in that the product of formula (I) is prepared, according to claim 1, in which A is H, $R_1$ is

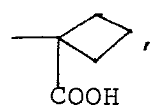

and $R_2$ is

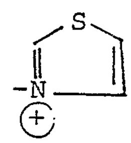

salified with $CF_3COO^-$.

4. Use of derivatives of the family of cephalosporins of formula (I) according to claim 1, for the preparation of an antibiotic drug.

**Patentansprüche** (für die Vertragsstaaten : BE, CH, DE, FR, GB, IT, LI, LU, NL, SE)

1. Derivate der Familie der Cephalosporine der Formel

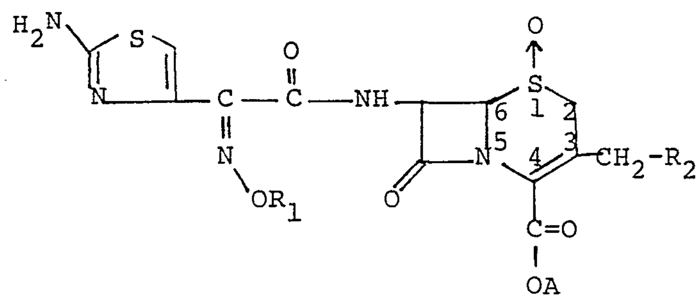

(I)

worin die Gruppe

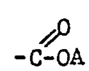

in der Position 4 ausgewählt ist aus Säureresten (A is H), den Alkali-, Erdalkali- oder Aminsalzen dieser Säure, wie Triäthylamin und den Äthanolaminen, und leicht hydrolysierbaren oder metabolisch labilen, pharmazeutisch akzeptablen Estergruppen,

— $R_1$ eine Gruppe

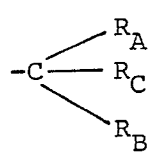

darstellt, worin $R_A$ und $R_B$ jeweils unabhängig Wasserstoff, eine Methylgruppe bezeichnen, oder $R_A$ und $R_B$, zusammen mit dem Kohlenstoffatom, an das sie gefunden sind, einen Cyclobutyl- oder Cyclopentylring bilden, und $R_C$ Wasserstoff oder eine Carboxylgruppe bedeutet,

— $R_2$ für die Gruppe

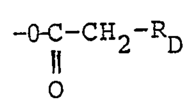

steht, worin $R_D$ einen 1,3-Thiazol-4-yl-ring darstellt, der gegebenenfalls in der Position 2 durch eine Aminogruppe substituiert ist, oder aber $R_2$ eine Thiazoliumgruppe bedeutet, die gegebenenfalls in der Position 2 durch eine Aminogruppe substituiert ist.

2. Derivate nach Anspruch 1, dadurch gekennzeichnet, daß sie die Form von syn- oder anti-Isomeren oder die Form einer Mischung dieser Isomeren aufweisen.

3. Verfahren zur Herstellung der Derivate nach Anspruch 1, worin A für H steht, dadurch gekennzeichnet, daß

— man als Ausgangsprodukt 7-Amino-3-brommethyl-3-cephem-4-tert.butyl-carboxylat-1-S-oxid, bei dem die Aminogruppe beispielsweise durch eine Tritylgruppe geschützt ist, verwendet,

— man das Produkt mit einer Säure der Formel

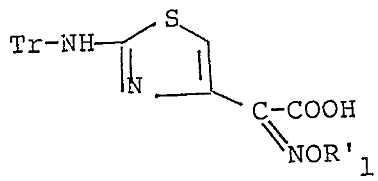

worin $R_1'$ $R_1$ oder ein tert.Butylester von $R_1$ ist, wenn $R_1$ eine Carboxylfunktion umfaßt, wobei die Säurefunktion der Säure vorzugsweise durch Überführen in das Anhydrid aktiviert worden ist, zur Umsetzung bringt, welche Umsetzung in einem Lösungsmittel bei einer Temperatur zwischen 0 und 50 °C, gegebenenfalls in Gegenwart eines hydroxylierten Derivats, wie 1-Hydroxy-benzotriazol, stattfindet,

— man das in Lösung erhaltene Produkt danach mit einem Derivat der Formel $R_2H$ zur Umsetzung bringt, wobei die Umsetzung in einem geeigneten Lösungsmittel in Gegenwart einer Base, wie Triäthylamin, stattfindet,

— und man dann die Schutzgruppen des erhaltenen Produkts entfernt.

4. Verfahren zur Herstellung der Produkte nach Anspruch 1, worin A ungleich H ist, dadurch gekennzeichnet, daß man unter Anwendung des Verfahrens nach Anspruch 3 ein Produkt nach Anspruch 1 herstellt und danach mittels bekannter Mittel den Wasserstoff der Säuregruppe in Position 4 umwandelt.

5. Als Antibiotika in der Human- und Veterinärmedizin nützliche Medikamente, dadurch gekennzeichnet, daß sie als Wirkstoff ein Produkt nach einem der Ansprüche 1 und 2 enthalten.

6. Medikamente nach Anspruch 5, dadurch gekennzeichnet, daß der verwendete Wirkstoff syn-Form aufweist.

7. Medikamente nach einem der Ansprüche 5 und 6, dadurch gekennzeichnet, daß das aktive Produkt ein Produkt der Formel (I) ist, worin A für H steht, $R_1$

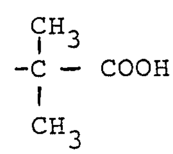

bedeutet und $R_2$

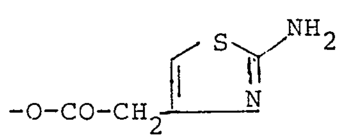

darstellt.

8. Medikament nach einem der Ansprüche 5 und 6, dadurch gekennzeichnet, daß das aktive Produkt ein Produkt der Formel (I) ist, worin A für H steht, $R_1$

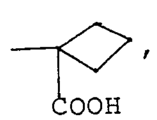

bedeutet und $R_2$ das Salz von

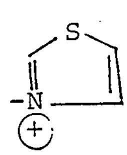

mit $CF_3COO^-$ darstellt.

**Patentansprüche** (für den Vertragsstaat AT)

1. Verfahren zur Herstellung von Derivaten der Familie der Cephalosprorine der Formel

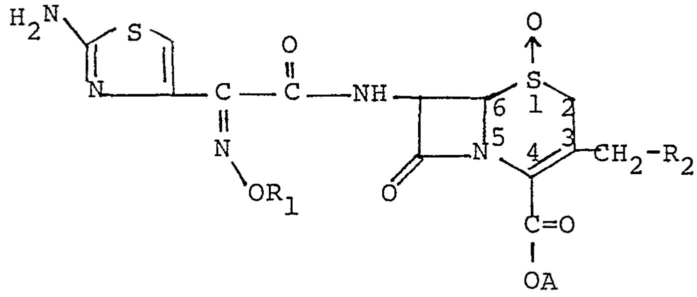

(I)

worin die Gruppe

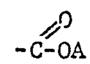

in der Position 4 ausgewählt ist aus Säureestern (A is H), den Alkali-, Erdalkali- oder Aminsalzen dieser Säure, wie Triäthylamin und den Äthanolaminen, und leicht hydrolysierbaren oder metabolisch labilen, pharmazeutisch akzeptablen Estergruppen,

— $R_1$ eine Gruppe

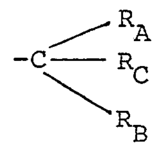

darstellt, worin $R_A$ und $R_B$ jeweils unabhängig Wasserstoff, eine Methylgruppe bezeichnen, oder $R_A$ und $R_B$, zusammen mit dem Kohlenstoffatom, an das sie gebunden sind, einen Cyclobutyl- oder Cyclopentylring bilden, und $R_C$ Wasserstoff oder eine Carboxylgruppe bedeutet,

— $R_2$ für die Gruppe

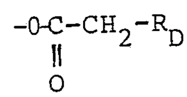

steht, worin $R_D$ einen 1,3-Thiazol-4-yl-ring darstellt, der gegebenenfalls in der Position 2 durch eine Aminogruppe substituiert ist, oder aber $R_2$ eine Thiazoliumgruppe bedeutet, die gegebenenfalls in der Position 2 durch eine Aminogruppe substituiert ist, welche Derivate in anti- oder syn-Form oder als Mischung dieser beiden Formen vorliegen, dadurch gekennzeichnet, daß

— man als Ausgangsprodukt 7-Amino-3-brommethyl-3-cephem-4-tert.butyl-carboxylat-1-S-oxid, bei dem die Aminogruppe beispielsweise durch eine Tritylgruppe geschützt ist, verwendet,

— man das Produkt mit einer Säure der Formel

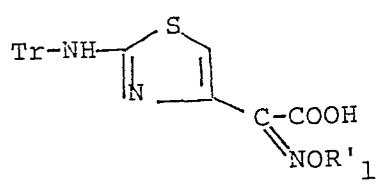

worin $R_1'$ $R_1$ oder ein tert.Butylester von $R_1$ ist, wenn $R_1$ eine Carboxylfunktion umfaßt, wobei die Säurefunktion der Säure vorzugsweise durch Überführen in das Anhydrid aktiviert worden ist, zur Umsetzung bringt, welche Umsetzung in einem Lösungsmittel bei einer Temperatur zwischen 0 und 50 °C, gegebenenfalls in Gegenwart eines hydroxylierten Derivats, wie 1-Hydroxy-benzotriazol, stattfindet,

— man das in Lösung erhaltene Produkt danach mit einem Derivat der Formel $R_2H$ zur Umsetzung bringt, wobei die Umsetzung in einem geeigneten Lösungsmittel in Gegenwart einer Base, wie Triäthylamin, stattfindet,

— und man dann die Schutzgruppen des erhaltenen Produkts entfernt, worauf man die Säure gegebenenfalls mittels bekannter Mittel in das Salz oder den Ester umwandelt.

2. Verfahren nach Anspruch 1, dadurch gekennzeichnet, daß man ein Produkt der Formel (I) herstellt, worin A für H steht, $R_1$

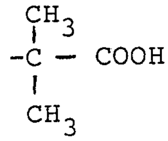

bedeutet und $R_2$

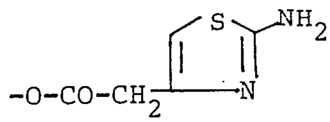

darstellt.

3. Verfahren nach Anspruch 1, dadurch gekennzeichnet, daß man ein Produkt der Formel (I) herstellt, worin A für H steht, $R_1$

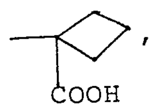

bedeutet und $R_2$ das Salz von

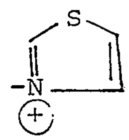

mit $CF_3COO^-$ darstellt.

4. Verwendung der Derivate der Familie der Cephalosporine der Formel (I) nach Anspruch 1 zur Herstellung eines Antibiotikums.